# EUROPEAN PATENT SPECIFICATION

(11) **EP 2 200 574 B1**
(45) Date of publication and mention of the grant of the patent: **26.06.2019**
(21) Application number: 08840361.3
(22) Date of filing: 17.10.2008
(51) Int. Cl.: A61K 8/35, A61Q 19/08, A61K 8/97

(54) **NOVEL USE OF PANDURATIN DERIVATIVES OR EXTRACT OF KAEMPFERIA PANDURATA COMPRISING THE SAME**
NEUE VERWENDUNG VON PANDURATIN-DERIVATEN ODER KAEMPFERIA PANDURATA-EXTRAKT DAMIT
UTILISATION NOUVELLE DE DÉRIVÉS DE PANDURATINE OU D'UN EXTRAIT DE KAEMPFERIA PANDURATA EN CONTENANT

(30) Priority: 17.10.2007 KR 20070104788
(43) Date of publication of application: 30.06.2010
(73) Proprietor: NEWTREE CO., LTD., Sangdaewon-Dong Joongwon-Gu Sungnam City Gyeonggi-Do 462-120 (KR)
(72) Inventor: HWANG, Jae-Kwan, Goyang-si Kyeonggi-do 412-270 (KR); SHIM, Jae-Seok, Goyang-si Kyeonggi-do 412-220 (KR); GWON, Song Hui, Bucheon-si Syeonggi-do 420-709 (KR); KWON, Yi Young, Anyang-si Gyeonggi-do 431-750 (KR); OH, Hyun In, Suwon-si Gyeonggi-do 440-832 (KR); HAN, Young Sun, Seoul 132-021 (KR); CHOI, Eun Jung, Seoul 137-937 (KR); KIM, Do Un, Gyeonggi-do 463-905 (KR)
(74) Representative: Isarpatent
(86) International application number: PCT/KR2008/006137
(87) International publication number: WO 2009/051434

(56) References cited:
- WO-A1-02/15860
- JP-A- 9 030 945
- JP-A- 2000 316 526
- JP-A- 2001 261 545
- JP-A- 2007 186 431
- KR-A- 20040 108 081
- SOHN, J. H. ET AL.: 'Protective effects of Panduratin A against Oxidative Damage of tert- butylhydroperoxide in human HepG2 cells' BIOL. PHARM. BULL. vol. 28, no. 6, 2005, pages 1083 - 6, XP008133096
- SHINDO, K. ET AL.: 'Analysis of antioxidant activities contained in the Boesenbergia pandurata Schult. Rhizome' BIOSCI. BIOTECHNOL. BIOCHEM. vol. 70, no. 9, 2006, pages 2281 - 4, XP008133098

## Description

### Technical Field

The present invention relates to a novel use of panduratin derivatives or a *Kaempferia pandurata* extract comprising the same in non-therapeutic methods.

### Background Art

Aging is largely classified into natural aging, or intrinsic aging, and extrinsic aging. Natural aging is caused by hereditary factors and is hard to control, whereas extrinsic aging is caused by environmental factors and can be controlled relatively easily. Accordingly, researches have continued to prevent extrinsic aging. Especially, researches on the prevention of wrinkles caused by extrinsic photoaging due to long-term exposure to UV are drawing attentions [Gilchre st B.A., J. Am. Acad. Dermatol., 1989: 21: 610-613]. The photoaging, or extrinsic skin aging, is clinically characterized by rough and inelastic skin, irregular pigmentation and deep wrinkles.

External factors that affect the aging include wind, temperature, humidity, cigarette smoke, pollution, UV, and the like. Especially, the aging caused by UV is called photoaging. Particularly, the photoaging is deeply involved in wrinkling on face and head, which are cosmetically important areas. Therefore, basic researches on photoaging and wrinkling on human skin or in animal model are actively carried out for the development of anti-aging or anti-wrinkling cosmetics. With regard to photoaging and wrinkling, changes in basic physiological metabolism such as synthesis and degradation of collagen, the main component of skin, are reported [Lavker R.M., Blackwell Science Inc.,1995:123-135].

The photoaging mechanism will be described briefly. When the skin is exposed to a large amount of UV, a lot of reactive oxygen species are generated in the skin, disrupting the enzymatic and non-enzymatic antioxidative defense system. As a result, the content of collagen, the main protein of the skin tissue, decreases remarkably. Collagenase (matrix metalloproteinase-1; MMP-1) plays an important role in the decrease of collagen. This enzyme is involved in the degradation of the extracellular matrix and basement membrane. According to researches, exposure to UV leads to increased MMP-1 activity in the skin, thereby markedly disrupting collagen and forming wrinkles [Sim G.S., Kim J.H., et al., Kor. J. Biotechnol. Bioeng., 2005:20 (1) :40-45].

Some of active ingredients for treating wrinkles and preventing aging, which have been developed to date, have problems in that they cannot be used as cosmetic materials, are very unstable and are not easy to deliver to the skin. Accordingly, a special stabilizing system and delivery system are required, and the effect thereof on treating skin wrinkles is not visible. For this reason, interest in skin-protecting agents containing retinoid has recently been increased. Currently, retinoid is used as a means for solving photoaging phenomena, such as wrinkles resulting from sunlight, skin thickening, skin drooping and a decrease in skin elasticity. However, retinoid has a problem in that it is a very unstable compound, which is sensitive to UV light, moisture, heat and oxygen such that a chemical change therein easily occurs. In attempts to solve this problem, studies focused on developing effective components derived from natural resources have been conducted.

*Kaempferia pandurata,* also known as *Boesenbergia pandurata,* is a plant of the Zingiberaceae family. Its rhizome is widely used to treat cold, enteritis, skin disease and urethral pain. *Kaempferia pandurata* contains pinocembrin chalcone, cardamonin, pinocembrin, pinostribin, 4-hydroxypanduratin A, panduratin A, and the like. These components are reported to have anti-cancer effect [Trakoontivakorn, G., et al., J. Agri. Food Chem., 49, 3046-3050, 2001], and flavonoid-based dihydrochalcone compounds are reported to have insecticidal effect [Pandji, C., et al., Phytochemistry, 34, 415-419, 1993]. Korean Patent No. 492034 discloses an antimicrobial and an oral composition for the prevention and treatment of caries and periodontitis comprising panduratin derivatives such as isopanduratin A. However, wrinkle treatment effect or anti-aging effect of panduratin derivatives or a *Kaempferia pandurata* extract comprising the same have never been reported yet.

JP2007186431 discloses a method for suppressing oxidation in the body, using *Boesenbergia rotunda (Kaempferia pandurata)* or 4-hydroxypanduratin in a medicinal or food composition. Ageing is mentioned very briefly.

JP2000316526 discloses a food composition for improving health, comprising extracts of *Boesenbergia pandurata.*

WO 02/15860 and KR 2004 0108081 disclose the use of antioxidant compounds in preventing/treating skin ageing.

Sohn, J. H. et al.: Biol. Pharm. Bull., vol. 28, no. 6, 2005, pages 1083-6 disclose the protective effects of panduratin A against oxidative damage to cells caused by t-butylhydroperoxide.

Shindo, K. et al. : Biosc. Biotechnol. Biochem., vol. 70, no. 9, 2006, pages 2281-4, disclose the antioxidant effect of extracts *of Boesenbergia pandurata (Kaempferia pandurata.*

JP2001261545 discloses a skin care preparation for the prevention of chapped skin symptoms due to diseases such as psoriasis, contact dermatitis etc., which comprises an extract of *Kaempferia angustifolia.*

JP09030945 discloses a dermal preparation comprising an extract of *Boesenbergia pandurata* for skin whitening and also to prevent pigmentation after sunburn.

### Disclosure

### Technical Problem

The inventors of the present invention have researched on natural substances having wrinkle treatment or anti-aging activity. They found out that panduratin derivatives or a *Kaempferia pandurata* extract comprising the same have such an activity and accomplished the present invention.

Accordingly, the present invention provides non-therapeutic methods for promoting collagen synthesis and for inhibiting collagen degradation, and also non-therapeutic uses of cosmetic compositions, and non-therapeutic uses of a food and non-therapeutic uses of nutritional supplements, all for preventing and treating wrinkles, or achieving anti-aging effects comprising the use of a panduratin derivative selected from the group consisting of the compounds represented by the following Chemical Formulas 1 to 3 or a *Kaempferia pandurata* extract comprising the same:

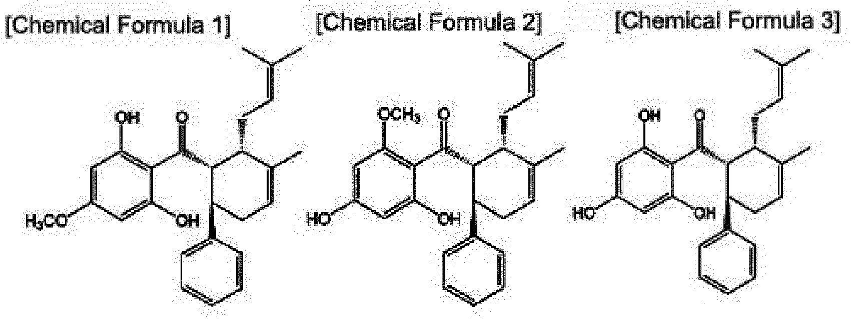

In one aspect, the present invention provides a non-therapeutic use of a cosmetic composition, a non-therapeutic use of a food and a non-therapeutic use of a nutritional supplement, all of which promote collagen synthesis, for preventing or treating wrinkle, or for preventing aging, using a panduratin derivative selected from the group consisting of the compounds represented by the Chemical Formulas 1 to 3 or a *Kaempferia pandurata* extract comprising the same.

In another aspect, the present invention provides a non-therapeutic use of a cosmetic composition, a food or a nutritional supplement, all of which inhibit collagen degradation, using a panduratin derivative selected from the group consisting of the compounds represented by the Chemical Formulas 1 to 3 or a *Kaempferia pandurata* extract comprising the same.

In another aspect, the present invention provides a non-therapeutic method for promoting collagen synthesis of a panduratin derivative selected from the group consisting of the compounds represented by the Chemical Formulas 1 to 3 or a *Kaempferia pandurata* extract comprising the same.

In another aspect, the present invention provides a non-therapeutic method for inhibiting collagen degradation of a panduratin derivative selected from the group consisting of the compounds represented by the Chemical Formulas 1 to 3 or a *Kaempferia pandurata* extract comprising the same.

### Technical Solution

To achieve the above objects, the present invention describes non-therapeutic uses of a cosmetic composition, of a food and of a nutritional supplement, and as well non-therapeutic methods for inhibiting collagen synthesis and for promoting collagen synthesis, each containing a panduratin derivative selected from the group consisting of the compounds represented by the following Chemical Formulas 1 to 3 or a *Kaempferia pandurata* extract comprising the same:

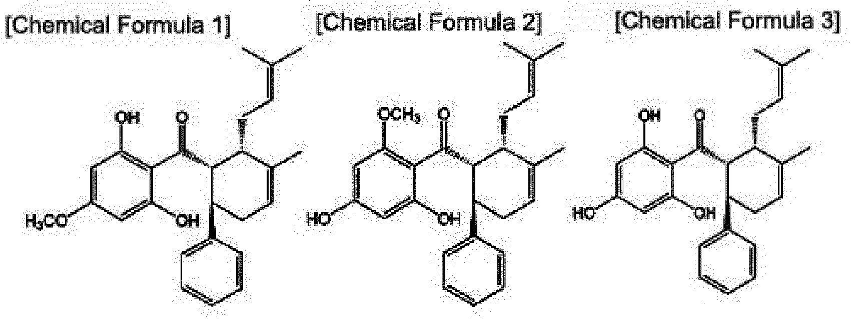

The present invention provides a non-therapeutic use of a cosmetic composition, a food or a nutritional supplement which promotes collagen synthesis for preventing or treating wrinkle, or for preventing aging, using a panduratin derivative selected from the group consisting of the compounds represented by the Chemical Formulas 1 to 3 or a *Kaempferia pandurata* extract comprising the same.

The present invention provides a non-therapeutic use of a cosmetic composition, a food or a nutritional supplement which inhibits collagen degradation for preventing or treating wrinkle, or for preventing aging, using a panduratin derivative selected from the group consisting of the compounds represented by the Chemical Formulas 1 to 3 or a *Kaempferia pandurata* extract comprising the same.

The present invention also provides a method for promoting collagen synthesis of a panduratin derivative selected from the group consisting of the compounds represented by the Chemical Formulas 1 to 3 or a *Kaempferia pandurata* extract comprising the same, thereby particularly preventing or treating wrinkle, or preventing aging.

The present invention further provides a method for inhibiting collagen degradation of a panduratin derivative selected from the group consisting of the compounds represented by the Chemical Formulas 1 to 3 or a *Kaempferia pandurata* extract comprising the same, thereby particularly preventing or treating wrinkle, or preventing aging.

Hereafter, the present invention will be described in detail.

The *"Kaempferia pandurata* extract" disclosed in the present invention refers to an extract obtained from *Kaempferia pandurata,* also known as *Boesenbergia pandurata,* comprising the aforesaid panduratin derivative. The method for preparing the *Kaempferia pandurata* extract is not specially limited as long as the panduratin derivative is included in the extract. Preferably, it may be prepared by extracting the whole plant or part (stem, rhizome or leaf) of *Kaempferia pandurata* (Roxb.) with at least one solvent selected from the group consisting of water, C₁-C₆ organic solvent and subcritical or supercritical fluid. If necessary, a process of filtration or condensation commonly used in the related art may be further added.

The C₁-C₆ organic solvent may be selected from C₁-C₆ alcohol, acetone, ether, benzene, chloroform, ethyl acetate, methylene chloride, hexane, cyclohexane and petroleum ether, but not limited thereto.

As used herein, the "supercritical fluid" refers to any substance at a temperature and pressure above its thermodynamic critical point. The "subcritical fluid" includes subcritical liquid and gas. Especially, the subcritical liquid refers to a fluid at temperatures below the supercritical temperature and the saturation temperature. And, the subcritical gas refers to a fluid at temperatures above the saturation temperature and pressures below the supercritical pressure. The supercritical fluid and subcritical fluid are used in various fields, including pharmaceutical industry, food industry, cosmetics/perfume industry, chemical industry and energy industry. The supercritical fluid and subcritical fluid that may be used in the present invention are not specially limited. For example, carbon dioxide, nitrogen, nitrous oxide, methane, ethylene, propane, propylene, petroleum ether, ethyl ether, cyclohexane, etc. may be used. Especially, carbon dioxide is preferred because it is easily available, relatively inexpensive, inexplosive, and sufficiently safe for processing. Carbon dioxide has a critical temperature of 31.1 °C and a critical pressure of 73.8 atm.

As an embodiment of the present invention, dry *Kaempferia pandurata* was ground, extracted using ethanol, hexane or chloroform solvent, filtered and concentrated to prepare an ethanol, hexane or chloroform extract of *Kaempferia pandurata.* Further, *Kaempferia pandurata* was added to a supercritical fluid extractor using carbon dioxide (CO₂) as supercritical fluid to prepare a supercritical extract of *Kaempferia pandurata* (see Example 1).

As used herein, the "panduratin derivative" refers to a compound selected from the group consisting of the compounds represented by the following Chemical Formulas 1 to 3. Specifically, the compounds represented by Chemical Formulas 1, 2 and 3 are panduratin A, isopanduratin A and 4-hydroxypanduratin A, respectively.

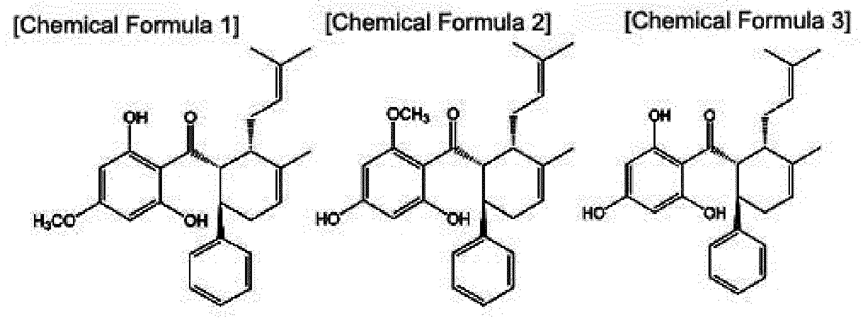

The panduratin derivative is commercially available or may be prepared according to a known synthesis method. It may be prepared by separating and purifying a *Kaempferia pandurata* extract or oil obtained by pressing *Kaempferia pandurata.* For the separation and purification of the panduratin derivative from the *Kaempferia pandurata* extract, column chromatography or high-performance liquid chromatography (HPLC) using silica gel, activated alumina or various other synthetic resins may be used alone or in combination, although not limited thereto.

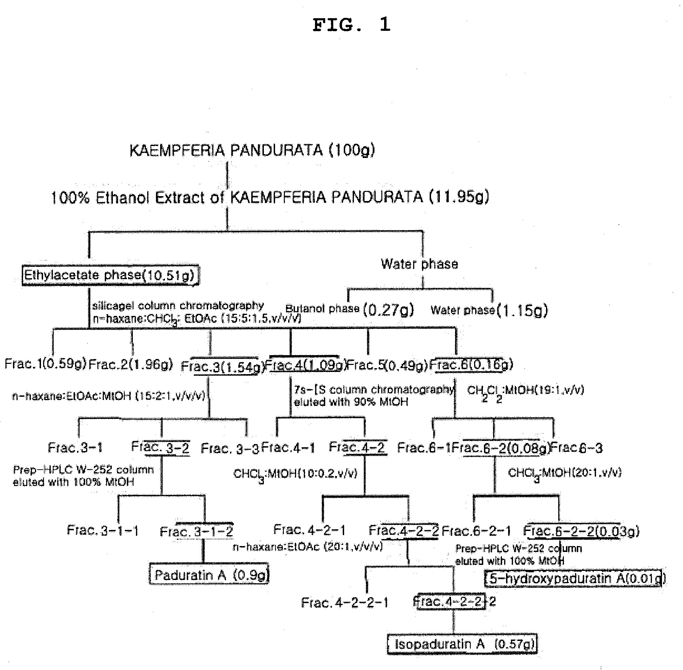

As an embodiment of the present invention, dry *Kaempferia pandurata* was ground and mixed with ethanol. After solvent extraction, the solvent was removed and the resulting extract was concentrated. The concentrated crude extract was mixed with ethyl acetate. After extracting the ethyl acetate soluble component followed by the removal of ethyl acetate, the ethyl acetate soluble component was concentrated and separated depending on polarities. Specifically, at first, development was carried out using a mixture solvent of hexane and ethyl acetate to remove impurities. Then, separation was carried out using a mixture solvent of hexane, chloroform and ethyl acetate. Finally, panduratin A, isopanduratin A or 4-hydroxypanduratin A was obtained (see Fig. 1 and Examples 2 through 4).

The panduratin derivative selected from the group consisting of the compounds represented by Chemical Formulas 1 to 3 or the *Kaempferia pandurata* extract comprising the same has superior anti-aging activity and is excellent in preventing or treating wrinkle.

Specifically, the panduratin derivative selected from the group consisting of the compounds represented by the Chemical Formulas 1 to 3 or the *Kaempferia pandurata* extract comprising the same has superior cell proliferation activity. When MTT assay was carried out using fibroblasts, the addition of the panduratin derivative selected from the group consisting of the compounds represented by the Chemical Formulas 1 to 3 or the *Kaempferia pandurata* extract comprising the same induced cell proliferation markedly (see Test Example 1).

Further, the panduratin derivative selected from the group consisting of the compounds represented by the Chemical Formulas 1 to 3 or the *Kaempferia pandurata* extract comprising the same has the activity of inhibiting collagen degradation by suppressing the expression of MMP-1, and promoting collagen synthesis by inducing the synthesis of procollagen. When treated with the panduratin derivative selected from the group consisting of the compounds represented by the Chemical Formulas 1 to 3 or the *Kaempferia pandurata* extract comprising the same, the expression of MMP-1 was suppressed whereas the procollagen synthesis increased (see Test Example 2).

The inventors have revealed the mechanism by which the panduratin derivative selected from the group consisting of the compounds represented by the Chemical Formulas 1 to 3 or the *Kaempferia pandurata* extract comprising the same inhibits collagen degradation and promotes collagen synthesis.

Specifically, collagen degradation occurs by the following mechanism. When extracellular-regulated protein kinase (ERK), Jun-N-terminal kinase (JNK) and p38 kinase, which belong to mitogen-activated protein kinases (MAPKs), are activated by phosphorylation, activation of activator protein-1 (AP-1) is induced [Xu Y, Fisher GJ., J Dermatol. Sci. Suppl. 2005; 1: S1 S8], resulting in binding with DNA. Through this, MMPs (matrix metalloproteinase) are excreted and collagen is degraded. Here, c-Jun and c-Fos are known to play a role in the binding of AP-1 with DNA [Waskiewicz AJ, Cooper JA., Curr. Opin. Cell Biol., 1995; 7: 798 805].

When panduratin A was added, the activity of ERK, JNK and p38 kinase, and the binding of AP-1 with DNA were suppressed. Further, the activity of c-Jun and c-Fos was suppressed. As a result, the secretion of MMPs was suppressed. Accordingly, panduratin A was confirmed to have the activity of inhibiting collagen degradation and promoting collagen synthesis (see Test Example 3).

Further, when applied on the skin or administered orally to mice in which wrinkling was induced by exposure to UV, the panduratin derivative selected from the group consisting of the compounds represented by the Chemical Formulas 1 to 3 or the *Kaempferia pandurata* extract comprising the same exhibited a remarkable wrinkle treatment effect (see Test Example 4).

Accordingly, the panduratin derivative selected from the group consisting of the compounds represented by the Chemical Formulas 1 to 3 or the *Kaempferia pandurata* extract comprising the same is excellent in preventing aging, and particularly, excellent in preventing, or treating wrinkle, thereby being used as an effective component of cosmetic, food or pharmaceutical composition. Claimed by the present invention are, however, only non-therapeutic methods for improving collagen synthesis and inhibiting collagen degradation and the use of non-therapeutic cosmetic compositions, a food or a nutritional supplement for inhibiting collagen degradation and for promoting collagen synthesis.

A composition for cosmetics not claimed per se contains the panduratin derivative selected from the group consisting of the compounds represented by the Chemical Formulas 1 to 3 or the *Kaempferia pandurata* extract comprising the same as an effective component, and may be prepared in the form of basic cosmetics (lotions, cream, essence, cleansers such as cleansing foam and cleansing water, pack, body oil), coloring cosmetics (foundation, lip-stick, mascara, make-up base), hair care composition (shampoo, rinse, hair conditioner, hair gel) and soap with dermatologically acceptable excipients.

The said excipients may comprise, but not limited thereto, skin softener, skin infiltration enhancer, colorant, odorant, emulsifier, thickener, or solvent. In addition, it is possible to add fragrance, a pigment, bactericidal agent, an antioxidant, a preservative, moisturizer and the like, and to add thickening agents, inorganic salts or synthetic polymers for improving physical properties. For example, in case of manufacturing a cleanser and soap comprising composition of the present invention, they may be prepared easily by adding the panduratin derivative or the *Kaempferia pandurata* extract comprising the same to conventional cleanser or soap base. In case of manufacturing a cream, it may be prepared by adding the panduratin derivative or the *Kaempferia pandurata* extract comprising the same to conventional oil-in-water cream base. In addition, it is possible to add a fragrance, a chelating agent, a pigment, an antioxidant, a preservative, and the like, and to add proteins, minerals or synthetic polymers for improving physical properties.

The panduratin derivative or the *Kaempferia pandurata* extract comprising the same of the present invention may be preferably comprised by the form of composition for cosmetics not claimed per se in the range of 0.001-10 wt%, and more preferably 0.01-5 wt%, based on the total weight of a formulation. If the composition is added in an amount of less than 0.001 wt%, it will provide low effect in preventing aging or treating wrinkle, and if it is added in an amount of more than 10 wt%, it will have a difficulty in safety or formulation.

A composition for food to be used in accordance with the invention may comprise all kinds of forms including functional food, nutritional supplement, health food, and food additives.

The said composition for food may be prepared into various kinds of forms by the methods known in the art.

For example, as a health food, the panduratin derivative or the *Kaempferia pandurata* extract comprising the same of the present invention may be prepared into tea, juice, and drink for drinking or may be prepared into granules, capsules, or powder for uptake. Also, conventional active ingredient which is well known as having activity in prevention of aging or prevention or treatment of wrinkle may be mixed with the panduratin derivative or the *Kaempferia pandurata* extract comprising the same of the present invention so as to prepare a composition. Also, for preparing functional foods, the panduratin derivative or the *Kaempferia pandurata* extract comprising the same of the present invention may be added to beverages (including alcoholic beverages), fruits, and their processed foods (e.g. canned fruit, bottled fruit, jam, marmalade etc.), fishes, meats, and their processed foods (e.g. ham, sausage, corn beef etc.), breads and noodles (e.g. Japanese noodle, buckwheat noodle, Chinese noodle, spaghetti, macaroni etc.), fruit juice, drinks, cookies, toffee, dairy products (e.g. butter, cheese etc.), vegetable oil, margarine, vegetable protein, retort food, frozen food, various seasonings (e.g. soybean paste, soybean sauce, sauce etc.). In addition, the panduratin derivative or the *Kaempferia pandurata* extract comprising the same may be prepared in a form of powder or extract for food additives.

The panduratin derivative or the *Kaempferia pandurata* extract comprising the same may be properly comprised by the form of composition for food preferably in the range of 0.01 to 50% based on the total weight of a food. More preferably, a food composition to be used in the invention may be prepared particularly by mixing conventional active ingredient which is well known as having activity in prevention of aging or prevention or treatment of wrinkle with the panduratin derivative or *Kaempferia pandurata* extract comprising the same of the present invention.

Further, because the panduratin derivative selected from the group consisting of the compounds represented by the Chemical Formulas 1 to 3 or the *Kaempferia pandurata* extract comprising the same has superior anti-aging activity and is especially excellent in preventing or treating wrinkle, it can be used for preparing a cosmetic composition, food composition or pharmaceutical composition for anti-aging, preventing or treating wrinkle. Said compositions are not claimed per se.

The method for preparing the cosmetic composition, food composition or pharmaceutical composition using the panduratin derivative or the *Kaempferia pandurata* extract is the same as described above. And, the content of the panduratin derivative or the *Kaempferia pandurata* extract in the compositions is the same as described above.

According to the present invention, the panduratin derivative selected from the group consisting of the compounds represented by the Chemical Formulas 1 to 3 or the *Kaempferia pandurata* extract comprising the same is used in a non-therapeutic cosmetic composition, a food or a nutritional supplement for method for preventing or treating wrinkle, or for preventing aging. When the non-therapeutic methods of the invention are used for preventing or treating wrinkle, or for preventing aging, the panduratin derivative or the *Kaempferia pandurata* extract may be administered to a subject in need thereof with an effective amount.

As used herein, the "subject in need" refers to a mammal, preferably a human, in need of preventing, or treating wrinkle, or preventing aging. And, the "effective amount" refers to an amount which exhibits the effect of preventing or treating wrinkle, and preventing aging by inhibiting collagen degradation and promoting collagen synthesis in the subject. The administration method and administration dose for administering with the effective amount are the same as described in detail above.

As described and claimed by the invention, the panduratin derivative selected from the group consisting of the compounds represented by the Chemical Formulas 1 to 3 or the *Kaempferia pandurata* extract comprising the same is also used in a non-therapeutic method for promoting collagen synthesis and inhibiting collagen degradation.

The activity of collagen synthesis promotion and collagen degradation inhibition of the panduratin derivative or the *Kaempferia pandurata* extract and the mechanism thereof are the same as described above.

### Advantageous Effects

As can be seen from the foregoing, the panduratin derivative selected from the group consisting of the compounds represented by the Chemical Formulas 1 to 3 or the *Kaempferia pandurata* extract comprising the same induces cell proliferation, inhibits degradation of collagen, and promotes synthesis of collagen; therefore, it shows excellent activity in preventing aging, particularly preventing or treating wrinkle.

### Description of Drawings

Fig. 1 illustrates a process of isolating the substances having wrinkle treatment activity from *Kaempferia pandurata.*
Fig. 2 shows MMP-1 inhibition activity and procollagen synthesis promotion activity of the ethanol extract of *Kaempferia pandurata* (A: MMP-1 expression inhibition activity, B: MMP-1 mRNA expression inhibition activity, C: procollagen biosynthesis promotion activity, D: procollagen mRNA expression promotion activity).
Fig. 3 shows MMP-1 inhibition activity and procollagen synthesis promotion activity of panduratin A (A: MMP-1 expression inhibition activity, B: MMP-1 mRNA expression inhibition activity, C: procollagen biosynthesis promotion activity, D: procollagen mRNA expression promotion activity).
Fig. 4 shows MMP-1 inhibition activity and procollagen synthesis promotion activity of isopanduratin A (A: MMP-1 expression inhibition activity, B: MMP-1 mRNA expression inhibition activity, C: procollagen biosynthesis promotion activity, D: procollagen mRNA expression promotion activity).
Fig. 5 shows MMP-1 inhibition activity and procollagen synthesis promotion activity of 4-hydroxypanduratin (A: MMP-1 expression inhibition activity, B: MMP-1 mRNA expression inhibition activity, C: procollagen biosynthesis promotion activity, D: procollagen mRNA expression promotion activity).
Fig. 6 shows the effect of the *Kaempferia pandurata* extract or the panduratin derivatives on activation of MAPKs.
Fig. 7 shows the effect of the *Kaempferia pandurata* extract or the panduratin derivatives on the DNA binding activity of AP-1.
Fig. 8 shows the effect of the *Kaempferia pandurata* extract or the panduratin derivatives on the c-Jun and c-Fos activity.
Fig. 9 shows skin replicas of mice after application of the *Kaempferia pandurata* extract or the panduratin derivatives.
Fig. 10 shows Rt, Rm, Rz and Ra measurement result after application of the *Kaempferia pandurata* extract or the panduratin derivatives on the skin of mice (Rt: the distance from the highest and lowest portions on the skin surface, Rm: the maximum Rt value of 5 measurements, Rz: the mean Rt value of 5 measurements, Ra: the arithmetic mean surface roughness).
Fig. 11 shows skin replicas of mice after oral administration of the *Kaempferia pandurata* extract or the panduratin derivatives.

### Mode for Invention

Hereinafter, the constitution and effect of the present invention will be described in detail through examples and test examples.

### Example 1: Preparation of Kaempferia pandurata extract comprising panduratin

### 1-1. Preparation of ethanol extract of Kaempferia pandurata

Dry *Kaempferia pandurata* rhizome was ground using a mixer. 100 g of the ground *Kaempferia pandurata* sample was added to 1 L of ethanol and extracted at room temperature for 48 hours. The extracted sample was filtered through Whatman No. 2 filter paper. The solvent component was removed from the filtered extract solution by concentrating using a vacuum rotary evaporator. An ethanol extract of *Kaempferia pandurata* was obtained.

### 1-2. Preparation of hexane extract of Kaempferia pandurata

Dry *Kaempferia pandurata* rhizome was ground using a mixer. 100 g of the ground *Kaempferia pandurata* sample was added to 1 L of hexane and extracted at room temperature for 48 hours. The extracted sample was filtered through Whatman No. 2 filter paper. The solvent component was removed from the filtered extract solution by concentrating using a vacuum rotary evaporator. A hexane extract of *Kaempferia pandurata* was obtained.

### 1-3. Preparation of chloroform extract of Kaempferia pandurata

Dry *Kaempferia pandurata* rhizome was ground using a mixer. 100 g of the ground *Kaempferia pandurata* sample was added to 1 L of chloroform and extracted at room temperature for 48 hours. The extracted sample was filtered through Whatman No. 2 filter paper. The solvent component was removed from the filtered extract solution by concentrating using a vacuum rotary evaporator. A chloroform extract of *Kaempferia pandurata* was obtained.

### 1-4. Preparation of supercritical extract of Kaempferia pandurata

A supercritical extract was obtained from *Kaempferia pandurata* rhizome using a supercritical fluid extractor and using carbon dioxide (CO₂) as supercritical fluid. After extracting at 50 °C and 200 bar, the solvent component was removed from the extract solution. A supercritical extract was obtained.

### Example 2: Isolation of panduratin A

The concentrated ethanol extract of *Kaempferia pandurata* obtained in Example 1-1 was mixed with ethyl acetate. The ethyl acetate soluble component was extracted and ethyl acetate was removed under reduced pressure to concentrate the ethyl acetate soluble component. Using a column in which silica gel was packed with 6 x 15 cm, and using a solvent system consisting of *n*-hexane, chloroform and ethyl acetate (15 : 5 : 1.5, v/v/v), a total of 6 fractions were concentrated and dried. Of the 6 fractions, the 3rd fraction (fraction 3) was subjected to thin layer chromatography (TLC, silica gel 60F254, Merck) using a developing solvent consisting of *n*-hexane, ethyl acetate and methanol (18 : 2 : 1, v/v/v). A total of 3 fractions were concentrated and dried. Of the 3 fractions, the 2nd fraction (fraction 3-2) was subjected to recycling HPLC (column: W-252, 20.0 mm ID x 500 mm L). A total of 2 fractions were concentrated and dried. Finally, of the 2 fractions, the 2nd fraction (fraction 3-2-2) was concentrated and dried. Panduratin A of the following Chemical Formula 1 was isolated as a pure substance having wrinkle treatment activity.

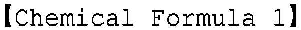

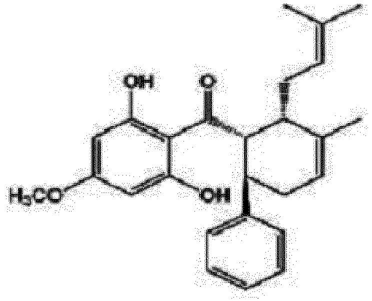

### Example 3: Isolation of isopanduratin A

The concentrated ethanol extract of *Kaempferia pandurata* obtained in Example 1-1 was mixed with ethyl acetate. The ethyl acetate soluble component was extracted and ethyl acetate was removed under reduced pressure to concentrate the ethyl acetate soluble component. Using a column in which silica gel was packed with 6 x 15 cm, and using a solvent system consisting of *n*-hexane, chloroform and ethyl acetate (15 : 5 : 1.5, v/v/v), a total of 6 fractions were concentrated and dried. Of the 6 fractions, the 4th fraction (fraction 4) was eluted using a reverse phase-18 (Rp-18, LiChropep, 25-40 m) packing material and using a solvent system consisting of methanol and water (9 : 1, v/v). A total of 2 fractions were obtained. Of the 2 fractions, the 2nd fraction (fraction 4-2) was concentrated, dried and eluted using a solvent system consisting of chloroform and methanol (10 : 0.2, v/v). A total of 2 fractions were concentrated and dried. Of the 2 fractions, the 2nd fraction (fraction 4-2-2) was eluted using a solvent system consisting of *n*-hexane and ethyl acetate (10 : 3, v/v). A total of 2 fractions were concentrated and dried. Finally, of the 2 fractions, the 2nd fraction (fraction 4-2-2-2) was concentrated and dried. Isopanduratin A of the following Chemical Formula 2 was isolated as a pure substance having wrinkle treatment activity.

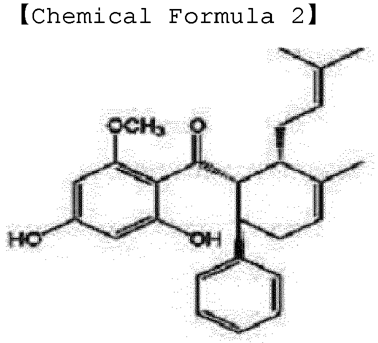

### Example 4: Isolation of 4-hydroxypanduratin A

The concentrated ethanol extract of *Kaempferia pandurata* obtained in Example 1-1 was mixed with ethyl acetate. The ethyl acetate soluble component was extracted and ethyl acetate was removed under reduced pressure to concentrate the ethyl acetate soluble component. Using a column in which silica gel was packed with 6 x 15 cm, and using a solvent system consisting of *n*-hexane, chloroform and ethyl acetate (15 : 5 : 1.5, v/v/v), a total of 6 fractions were concentrated and dried. Of the 6 fractions, the 6th fraction (fraction 6) was eluted using a solvent system consisting of methylene chloride and methanol (19 : 1, v/v). A total of 3 fractions were obtained. Of the 3 fractions, the 2nd fraction (fraction 6-2) was eluted using a solvent system consisting of chloroform and methanol (20 : 1, v/v). A total of 2 fractions were obtained. Finally, of the 2 fractions, the 2nd fraction (fraction 6-2-2) was subjected to recycling HPLC (column: W-252, 20.0 mm ID× 500 mm L). 4-Hydroxypanduratin A of the following Chemical Formula 3 was isolated as a pure substance having wrinkle treatment activity.

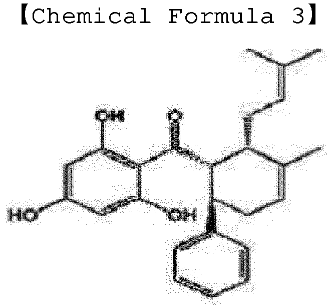

### Test Example 1: Cell proliferation effect of Kaempferia pandurata extract and panduratin derivatives

### 1-1. Cell proliferation of Kaempferia pandurata extract

MTT assay [3-(4,5-dimethylthiazol-2-yl)-2,5-diphenyltetrazolium bromide reduction method] was performed on fibroblasts to evaluate cell proliferation of the *Kaempferia pandurata* ethanol extract prepared in Example 1-1. A green tea extract, which is known to have skin wrinkle treatment effect, was selected as control substance. The result is given in the following Table 1.

**Table 1**

| Cell proliferation effect of *Kaempferia pandurata* extract | | |
|---|---|---|
| | Proliferation effect (%) | Proliferation effect (%) |
| Concentration (µg/mL) | *Kaempferia pandurata* extract | Green tea extract |
| 0 | 100 | 100 |
| 0.001 | 102 | 101 |
| 0.01 | 103 | 102 |
| 0.1 | 107 | 105 |
| 1 | 121 | 119 |
| 10 | 128 | 121 |
| 50 | 134 | 128 |

As seen from Table 1, the *Kaempferia pandurata* extract of the present invention exhibited better cell proliferation effect than the control substance.

### 1-2. Cell proliferation of panduratin derivatives

MTT assay [3-(4,5-dimethylthiazol-2-yl)-2,5-diphenyltetrazolium bromide reduction method] was performed on fibroblasts to evaluate cell proliferation of the panduratin derivatives prepared in Examples 2 through 4. Epigallocatechin-3-O-gallate (EGCG), which is known to have skin wrinkle treatment effect, was selected as control substance. The result is given in the following Table 2.

**Table 2**

| Cell proliferation effect of panduratin derivatives | | | | |
|---|---|---|---|---|
| | Proliferation effect (%) | Proliferation effect (%) | Proliferation effect (%) | Proliferation effect (%) |
| Concentration (µM) | Panduratin A | Isopanduratin A | 4-Hydroxypanduratin A | EGCG |
| 0 | 100 | 100 | 100 | 100 |
| 0.001 | 103 | 102 | 101 | 102 |
| 0.01 | 104 | 102 | 102 | 104 |
| 0.1 | 111 | 110 | 109 | 107 |
| 1 | 120 | 120 | 119 | 119 |
| 10 | 126 | 124 | 121 | 120 |
| 50 | 131 | 130 | 128 | 126 |

As seen from Table 2, the panduratin derivatives of the present invention exhibited better cell proliferation effect than the control substance.

### Test Example 2: MMP-1 inhibition and procollagen synthesis promotion effect of Kaempferia pandurata extract or panduratin derivatives

### 2-1. Kaempferia pandurata extract

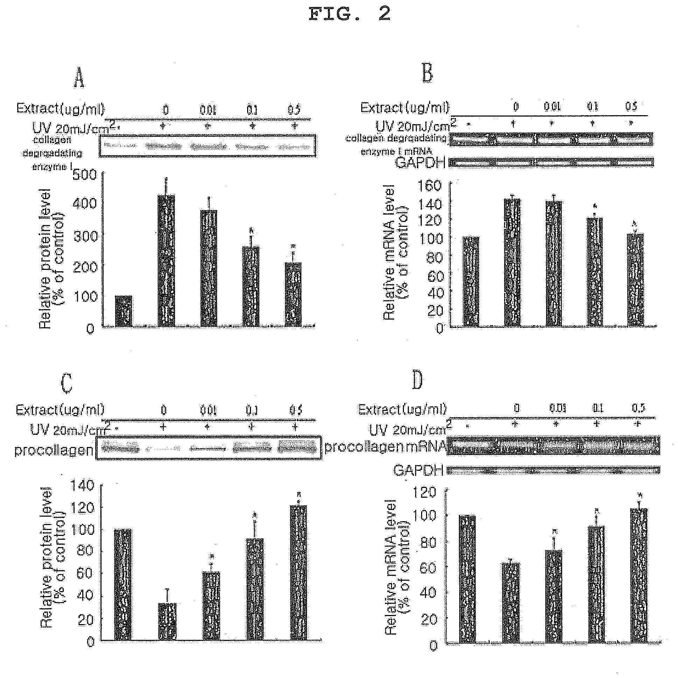

MMP-1 inhibition activity and procollagen synthesis promotion effect of the *Kaempferia pandurata* ethanol extract prepared in Example 1 were measured by Western blotting and reverse transcriptase-polymerase chain reaction (RT-PCR). The result is given in Fig. 2.

Specifically, proteins were extracted from cultured fibroblasts and quantitated using a protein assay reagent (Bio-Rad Laboratories Inc., Hercules, CA, USA). For Western blotting, the proteins were heated for 3 minutes and, after cooling, transferred to a nitrocellulose membrane (Amersham International, Little Chalfont, England) through electrophoresis in 10% SDS-PAGE. The membrane was saturated with 5 % skim milk in TBST (10 mM Tris, pH 7.5, 100 mM NaCl, 0.1% Tween 20). After blotting for 2 hours using primary antibodies (diluted to 1:1000), followed by washing with TBST, blotting was performed for 2 hours using secondary antibodies (diluted to 1:2000). After washing with TBST for 3 times, the blotted antibodies were analyzed using an ECL detection system (Amersham International, Little Chalfont, England).

For RT-PCR, RNAs were isolated from fibroblasts using TRIZOL (Invitrogen, USA). The isolated RNAs were quantitated using MMP-1 and procollagen primers and Taq polymerase. Specifically, RT-PCR was carried out using MMP-1 primer for 25 cycles, each cycle consisting of 30 seconds at 94 °C, 1 minute at 50 °C and 1 minute at 72 °C. And RT-PCR was carried out using procollagen primer for 28 cycles, each cycle consisting of 30 seconds at 94 °C, 1 minute at 60 °C and 1 minute at 72 °C. Then, after electrophoresis using 1% agarose gel, expression of mRNA for MMP-1 and procollagen was analyzed through ethidium bromide (EtBr) illumination.

As seen from Fig. 2, treatment with the *Kaempferia pandurata* ethanol extract resulted in the decrease of expression of MMP-1 proteins and mRNAs in a concentration-dependent manner, whereas expression of procollagen proteins and mRNAs increased in a concentration-dependent manner (see Fig. 2).

The same experiments were carried out for the hexane extract, chloroform extract and supercritical extract of *Kaempferia pandurata* prepared in Example 1. Treatment with the *Kaempferia pandurata* hexane extract resulted in 37% decrease of MMP-1 expression and 250% increase of procollagen synthesis as compared to the control group (not shown in the figure). And, treatment with the *Kaempferia pandurata* chloroform extract resulted in 40% decrease of MMP-1 expression and 290% increase of procollagen synthesis as compared to the control group (not shown in the figure). At last, treatment with the *Kaempferia pandurata* supercritical extract resulted in 29% decrease of MMP-1 expression and 220% increase of procollagen synthesis as compared to the control group (not shown in the figure).

Thus, it was confirmed that the *Kaempferia pandurata* extract can be effectively used for anti-aging or wrinkle treatment because it inhibits the expression of collagenase and promotes procollagen synthesis.

### 2-2. Panduratin derivatives

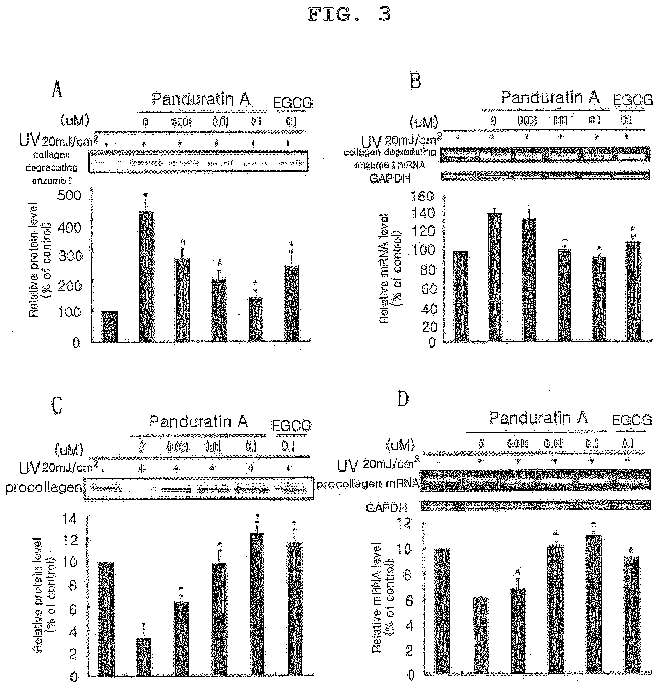

MMP-1 inhibition activity and procollagen synthesis promotion effect of the panduratin derivatives prepared in Examples 2 through 4 were measured by Western blotting and RT-PCR in the same manner as Test Example 2-1. The result is given in Figs. 3-5.

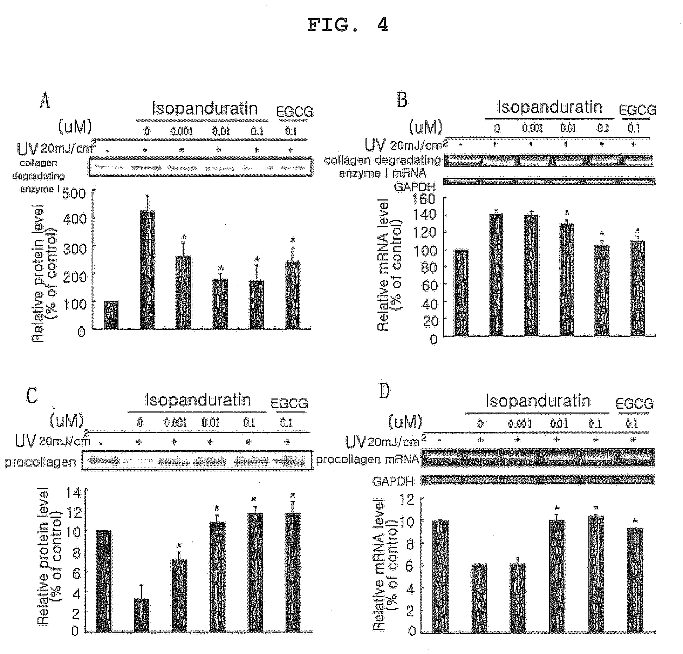

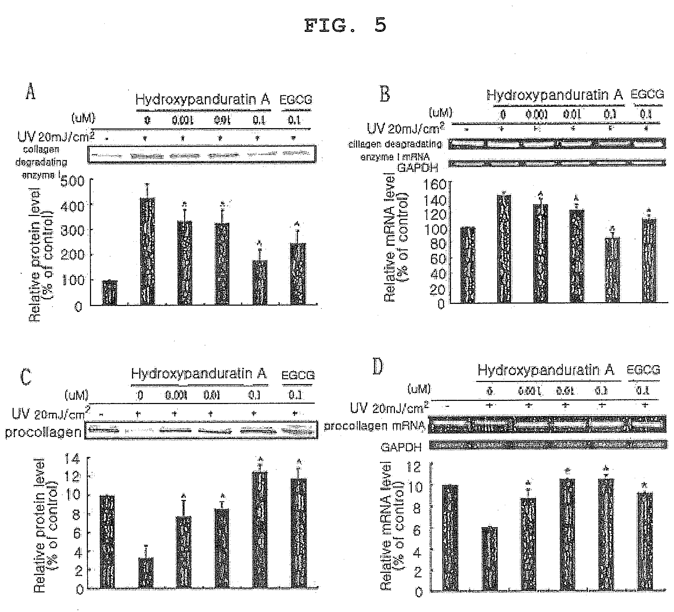

As seen from Figs. 3-5, treatment with panduratin A (see Fig. 3), isopanduratin A (see Fig. 4) or 4-hydroxypanduratin A (see Fig. 5) resulted in the decrease of MMP-1 proteins and mRNAs in a concentration-dependent manner, whereas expression of procollagen proteins and mRNAs increased in a concentration-dependent manner. Particularly, they exhibited better activity than the control substance EGCG.

Thus, it was confirmed that the panduratin derivatives can be effectively used for anti-aging or wrinkle treatment because they inhibit the expression of collagenase and promotes procollagen synthesis.

### Test Example 3: Collagen degradation inhibition mechanism of Kaempferia pandurata extract or panduratin derivatives

### 3-1. Effect on activation of mitogen-activated protein kinases (MAPKs)

Human skin fibroblasts (CCD-986sk, ATCC, Manassas, VA, USA) were cultured in DMEM (Gibco, Grand Island, NY, USA). The fibroblasts were cultured on a 10 cm Petri dish (SPL, Seoul, Korea) to a concentration of 80% and further cultured for 24 hours in serum-free culture medium. Then, the cells were cultured for 24 hours in serum-free DMEM containing the panduratin A of Example 2. After replacing the culture medium with 5 mL of phosphate-buffered saline (PBS), the cells were exposed to UV (20 mJ/cm²). Cells that were not exposed to UV were used as negative control group, and cells treated with EGCG were used as positive control group.

The fibroblasts were lysed in RIPA buffer (Sigma-Aldrich Co., St. Louis, MO, USA) and proteins were quantitated using a protein assay reagent (Bio-Rad Laboratories Inc., Hercules, CA, USA). For Western blotting, the proteins were heated for 3 minutes and, after cooling, transferred to a nitrocellulose membrane (Amersham International, Little Chalfont, England) through electrophoresis in 10% SDS-PAGE. The membrane was saturated with 5 % skim milk in TBST (10 mM Tris, pH 7.5, 100 mM NaCl, 0.1% Tween 20). After blotting for 2 hours using primary antibodies (diluted to 1:1000), followed by washing with TBST, blotting was performed for 2 hours using secondary antibodies (diluted to 1:2000). After washing with TBST for 3 times, the blotted antibodies were analyzed using an ECL detection system (Amersham International, Little Chalfont, England). The detection level was measured using the software RFLPscan version 2.1.

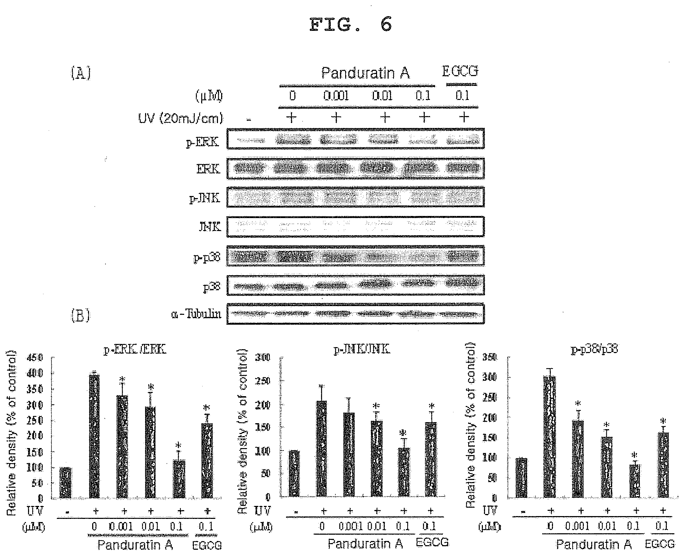

Through this procedure, the change of activity of extracellular-regulated protein kinase (ERK), Jun-N-terminal kinase (JNK) and p38 kinase, which belong to the MAPKs, was measured. The result is given in Fig. 6.

As seen from Fig. 6, ERK, JNK and p38 kinase were activated by phosphorylation when exposed to UV. But, the activation of ERK, JNK and p38 kinase was suppressed in a concentration-dependent manner when panduratin A was added. Considering that the activation of ERK, JNK and p38 kinase by phosphorylation induces the activation of activator protein-1 (AP-1) [Xu Y, Fisher GJ., J. Dermatol. Sci. Suppl. 2005; 1: S1 S8], thereby promoting secretion of matrix metalloproteinases (MMPs) and degradation of collagen [Huang C, Schmid PC, Ma WY, Schmid HH, Dong Z., J. Biol. Chem. 1997; 272: 4187 94], panduratin A may be effectively used for anti-aging or wrinkle treatment through inhibition of collagen degradation.

### 3-2. Effect on DNA binding activity of AP-1

Electrophoretic mobility shift assay (EMSA) was performed to measure the DNA binding activity of AP-1. Specifically, the fibroblasts, which were cultured in Test Example 3-1, to which panduratin A was added and which were exposed to UV, were washed with PBS and collected. After resuspending them in 100 µL of lysis buffer (10 mM HEPES, 10 mM KCl, 0.1 mM EDTA, 1 mM DTT, 0.5 mM PMSF, pH 7.9) for 15 minutes, 30 µL of 5% NP-40 was added and mixed for 15 seconds. The cytosol component was removed by centrifuge and nuclear pellets were lysed using extraction buffer (20 mM HEPES, 0.4 M NaCl, 1 mM EDTA, 1 mM DTT, 1 mM PMSF, pH 7.9). Gel shift assay was carried out using quantitated nuclear proteins according to the manufacturer's instructions (Gel Shift Kit System; Panomics, Fremont, CA, USA). Then, the detected proteins were analyzed with an ECL detection system (Amersham International, Little Chalfont, England) and the detection level was measured using the software RFLPscan version 2.1.

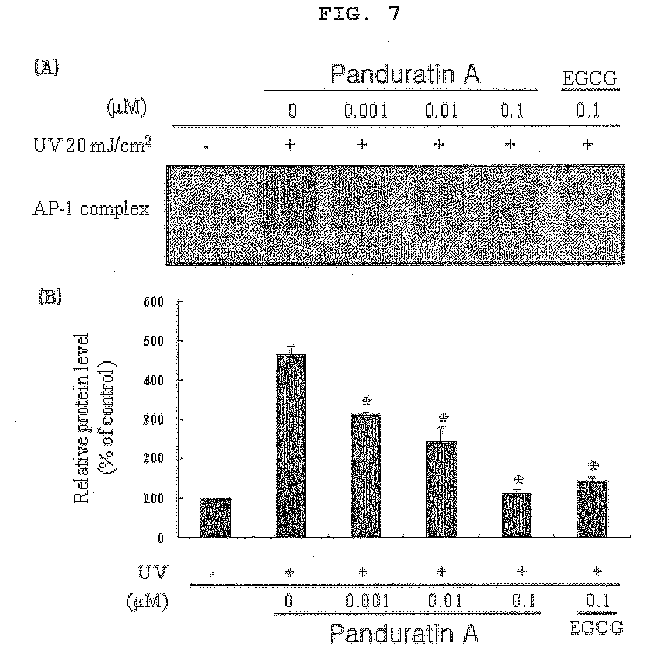

DNA binding activity of AP-1 was determined through this procedure. The result is given in Fig. 7.

As seen from Fig. 7, the addition of panduratin A resulted in inhibited binding of AP-1 to DNA in a concentration-dependent manner. Accordingly, considering that the activation of AP-1 may promote the secretion of MMPs and degradation of collagen, panduratin A may be effectively used for anti-aging or wrinkle treatment through inhibition of collagen degradation.

### 3-3. Effect on c-Jun and c-Fos activity

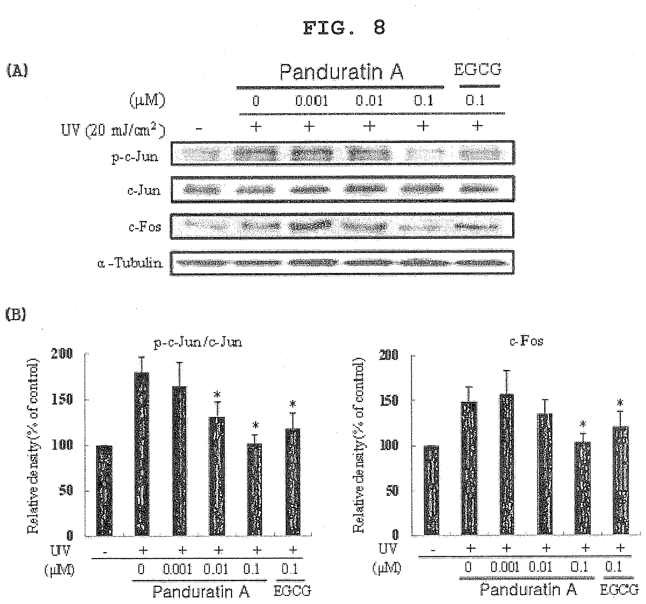

Western blotting was performed in the same manner as in Test Example 3-1 in order to investigate the effect of panduratin A on c-Jun and c-Fos activity. The result is given in Fig. 8.

As seen from Fig. 8, the addition of panduratin A resulted in inhibited c-Jun and c-Fos activity in a concentration-dependent manner. Considering the effect of c-Jun and c-Fos on transcriptional activity of AP-1 [Waskiewicz AJ, Cooper JA., Curr. Opin. Cell Biol., 1995; 7: 798 805], it can be seen that the panduratin A may inhibit AP-1 activity, as in Test Example 3-2.

### Test Example 4: Wrinkle treatment effect of Kaempferia pandurata extract or panduratin derivatives(not claimed per se)

### 4-1. Application on skin

Forty eight 6-week-old female hairless mice (Hos: HR-1) were accustomed for a week and randomly divided into 6 groups, 8 per each. The hairless mice (Hos:HR-1) were exposed to UV for 8 weeks. UV irradiation was carried out 3 times a week, from 1 MED (1 MED = 50 mJ/cm²) to 4 MED, until the end of the test. The 6 test groups were: non UV-treated group, UV-treated group, UV-and *Kaempferia pandurata* ethanol extract (0.1%, 0.5%)-treated group, and UV- and panduratin A (1 mM, 5 mM)-treated group. Each sample was dissolved in a mixture solvent of ethanol and polyethylene glycol (7:3, v/v) and 50 µL was applied on the back of the mice every day for 8 weeks. For the non UV-treated group and the UV-treated group, 50 µL of a mixture of ethanol and polyethylene glycol (7:3, v/v) was applied.

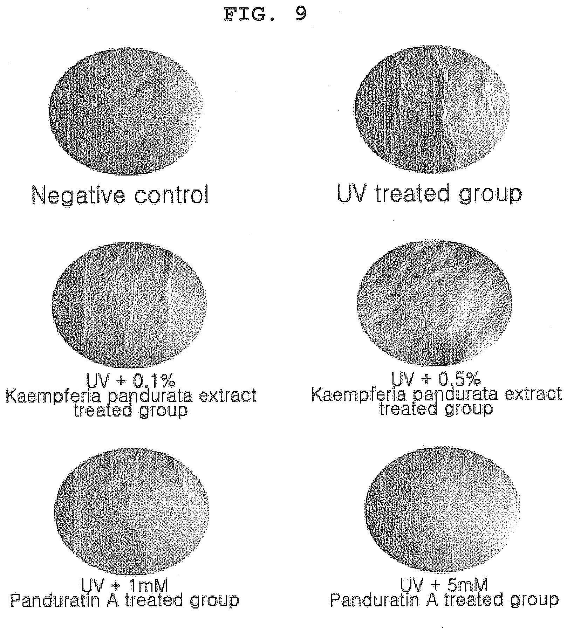

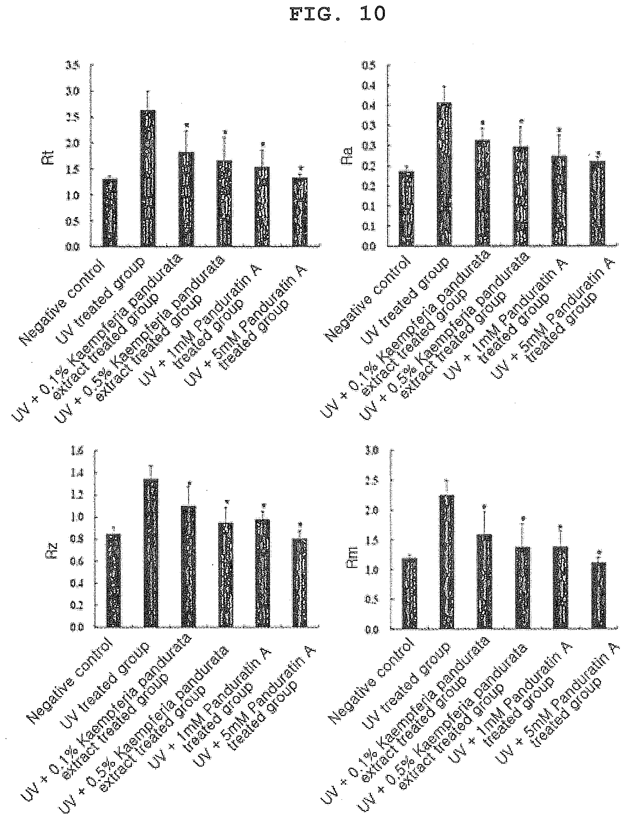

In order to investigate wrinkle prevention effect, skin replicas were taken using silicone polymer (SILFLO Impression Material, Flexico, England). The image files of the skin replicas were subjected to wrinkle evaluation using the computer image analysis software Skin Visiometer SV 600 (Courage+Khazaha Electronic, Kln, Germany). Rt, Rm, Rz and Ra values (Rt: the distance from the highest and lowest portions on the skin surface, Rm: the maximum Rt value of 5 measurements, Rz: the mean Rt value of 5 measurements, Ra: the arithmetic mean surface roughness) were determined. The result is given in Figs. 9 and 10.

As seen from Fig. 9, the *Kaempferia pandurata* ethanol extract-treated group (0.1 % and 0.5 %) and the panduratin A-treated group (1 mM and 5 mM) exhibited remarkably decreased wrinkling as compared to the UV-treated group. Further, as seen from Fig. 10, both the *Kaempferia pandurata* ethanol extract-treated group and the panduratin A-treated group exhibited significantly decreased Rt, Rm, Rz and Ra values (p < 0.05).

Accordingly, it can be seen that the application of the *Kaempferia pandurata* extract or the panduratin derivative on skin provides excellent wrinkle treatment effect.

### 4-2. Oral administration

To the hairless mice exposed to UV in Test Example 4-1, the *Kaempferia pandurata* ethanol extract (200 mg/kg) or panduratin A (50 mg/kg) dissolved in 0.5 % carboxymethyl cellulose solution containing 5 % Tween 80 was orally administered every day for 8 weeks. For the control groups (non UV-treated group and UV-treated group), 0.5 % carboxymethyl cellulose solution was administered.

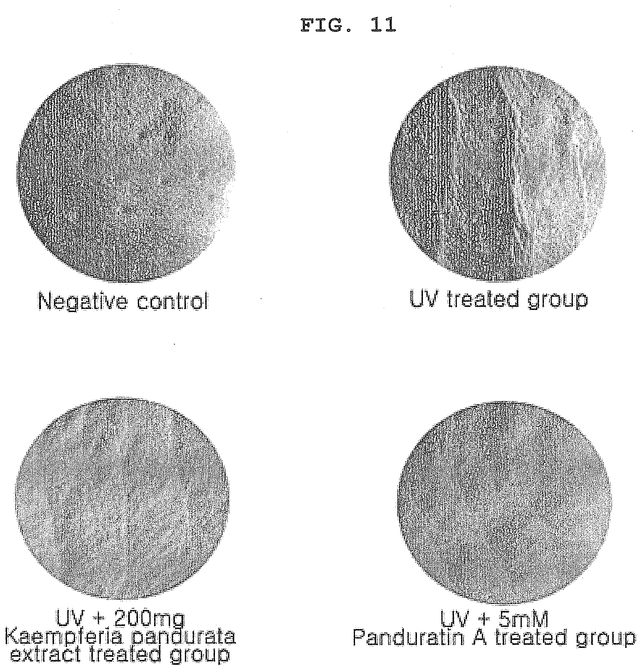

In order to investigate wrinkle prevention effect, skin replicas were taken using silicone polymer (SILFLO Impression Material, Flexico, England). The result is given in Fig. 11.

As seen from Fig. 11, the oral administration of the *Kaempferia pandurata* extract or the panduratin derivative exhibited remarkably decreased wrinkling.

Thus, it can be seen that the oral administration of the *Kaempferia pandurata* extract or the panduratin derivative provides excellent wrinkle treatment effect.

### Formulation Example 1: Cosmetics (not claimed per se) 1-1 and 1-2. Nourishing lotion (milk lotion)

Nourishing lotion was prepared according to a method commonly used in the related art using the panduratin derivative of Examples 2 through 4 or the *Kaempferia pandurata* extract of Example 1, with the compositions of the following Table 3.

**Table 3**

| Nourishing lotion (milk lotion) | | |
|---|---|---|
| | Formulation Example 1-1 (wt%) | Formulation Example 1-2 (wt%) |
| Panduratin derivative | 2.0 | - |
| *Kaempferia pandurata* extract | - | 2.0 |
| Squalene | 5.0 | 5.0 |
| Beeswax | 4.0 | 4.0 |
| Polysorbate 60 | 1.5 | 1.5 |
| Sorbitan sesquioleate | 1.5 | 1.5 |
| Liquid paraffin | 0.5 | 0.5 |
| Caprylic/capric triglyceride | 5.0 | 5.0 |
| Glycerine | 3.0 | 3.0 |
| Butylene glycol | 3.0 | 3.0 |
| Propylene glycol | 3.0 | 3.0 |
| Carboxyvinyl polymer | 0.1 | 0.1 |
| Triethanolamine | 0.2 | 0.2 |
| Antiseptic, pigment and perfume | adequate | adequate |
| Purified water | to 100 | to 100 |

### 1-3 and 1-4. Softening lotion (skin lotion)

Softening lotion was prepared according to a method commonly used in the related art using the panduratin derivative of Examples 2 through 4 or the *Kaempferia pandurata* extract of Example 1, with the compositions of the following Table 4.

**Table 4**

| Softening lotion (skin lotion) | | |
|---|---|---|
| | Formulation Example 1-3 (wt%) | Formulation Example 1-4 (wt%) |
| Panduratin derivative | 2.0 | - |
| *Kaempferia pandurata* extract | - | 2.0 |
| Glycerine | 3.0 | 3.0 |
| Butylene glycol | 2.0 | 2.0 |
| Propylene glycol | 2.0 | 2.0 |
| Carboxyvinyl polymer | 0.1 | 0.1 |
| PEG 12 nonylphenyl ether | 0.2 | 0.2 |
| Polysorbate 80 | 0.4 | 0.4 |
| Ethanol | 10.0 | 10.0 |
| Triethanolamine | 0.1 | 0.1 |
| Antiseptic, pigment and perfume | adequate | adequate |
| Purified water | to 100 | to 100 |

### 1-5 and 1-6. Nourishing cream

Nourishing cream was prepared according to a method commonly used in the related art using the panduratin derivative of Examples 2 through 4 or the *Kaempferia pandurata* extract of Example 1, with the compositions of the following Table 5.

**Table 5**

| Nourishing cream | | |
|---|---|---|
| | Formulation Example 1-5 (wt%) | Formulation Example 1-6 (wt%) |
| Panduratin derivative | 2.0 | - |
| *Kaempferia pandurata* extract | - | 2.0 |
| Polysorbate 60 | 1.5 | 1.5 |
| Sorbitan sesquioleate | 0.5 | 0.5 |
| PEG 60 hydrogenated castor oil | 2.0 | 2.0 |
| Liquid paraffin | 10 | 10 |
| Squalene | 5.0 | 5.0 |
| Caprylic/capric triglyceride | 5.0 | 5.0 |
| Glycerine | 5.0 | 5.0 |
| Butylene glycol | 3.0 | 3.0 |
| Propylene glycol | 3.0 | 3.0 |
| Triethanolamine | 0.2 | 0.2 |
| Antiseptic | adequate | adequate |
| Pigment | adequate | adequate |
| Perfume | adequate | adequate |
| Purified water | to 100 | to 100 |

### 1-7 and 1-8. Massage cream

Massage cream was prepared according to a method commonly used in the related art using the panduratin derivative of Examples 2 through 4 or the *Kaempferia pandurata* extract of Example 1, with the compositions of the following Table 6.

**Table 6**

| Massage cream | | |
|---|---|---|
| | Formulation Example 1-7 (wt%) | Formulation Example 1-8 (wt%) |
| Panduratin derivative | 1.0 | - |
| *Kaempferia pandurata* extract | - | 1.0 |
| Beeswax | 10.0 | 10.0 |
| Polysorbate 60 | 1.5 | 1.5 |
| PEG 60 hydrogenated castor oil | 2.0 | 2.0 |
| Sorbitan sesquioleate | 0.8 | 0.8 |
| Liquid paraffin | 40.0 | 40.0 |
| Squalene | 5.0 | 5.0 |
| Caprylic/capric triglyceride | 4.0 | 4.0 |
| Glycerine | 5.0 | 5.0 |
| Butylene glycol | 3.0 | 3.0 |
| Propylene glycol | 3.0 | 3.0 |
| Triethanolamine | 0.2 | 0.2 |
| Antiseptic, pigment and perfume | adequate | adequate |
| Purified water | to 100 | to 100 |

### 1-9 and 1-10. Pack

Pack was prepared according to a method commonly used in the related art using the panduratin derivative of Examples 2 through 4 or the *Kaempferia pandurata* extract of Example 1, with the compositions of the following Table 7.

**Table 7**

| Pack | | |
|---|---|---|
| | Formulation Example 1-9 (wt%) | Formulation Example 1-10 (wt%) |
| Panduratin derivative | 1.0 | - |
| *Kaempferia pandurata* extract | - | 1.0 |
| Polyvinyl alcohol | 13.0 | 13.0 |
| Sodium carboxymethyl cellulose | 0.2 | 0.2 |
| Glycerine | 5.0 | 5.0 |
| Allantoin | 0.1 | 0.1 |
| Ethanol | 6.0 | 6.0 |
| PEG 12 nonylphenyl ether | 0.3 | 0.3 |
| Polysorbate 60 | 0.3 | 0.3 |
| Antiseptic, pigment and perfume | adequate | adequate |
| Purified water | to 100 | to 100 |

### 1-11 and 1-12. Gel

Gel was prepared according to a method commonly used in the related art using the panduratin derivative of Examples 2 through 4 or the *Kaempferia pandurata* extract of Example 1, with the compositions of the following Table 8.

**Table 8**

| Gel | | |
|---|---|---|
| | Formulation Example 1-11 (wt%) | Formulation Example 1-12 (wt%) |
| Panduratin derivative | 0.5 | - |
| *Kaempferia pandurata* extract | - | 0.5 |
| Ethylenediamine sodium acetate | 0.05 | 0.05 |
| Glycerine | 5.0 | 5.0 |
| Carboxyvinyl polymer | 0.3 | 0.3 |
| Ethanol | 5.0 | 5.0 |
| PEG 60 hydrogenated castor oil | 0.5 | 0.5 |
| Triethanolamine | 0.3 | 0.3 |
| Antiseptic, pigment and perfume | adequate | adequate |
| Purified water | to 100 | to 100 |

### Formulation Example 2: Foods(not claimed per se) 2-1. Health food

1,000 mg of the panduratin derivative of Examples 2 through 4 or the *Kaempferia pandurata* extract of Example 1 may be mixed with 70 µg of vitamin A acetate, 1.0 mg of vitamin E, 0.13 mg of vitamin B₁, 0.15 mg of vitamin B₂, 0.5 mg of vitamin B₆, 0.2 µg of vitamin B₁₂, 10 mg of vitamin C, 10 µg of biotin, 1.7 mg of nicotinamide, 50 µg of folic acid, 0.5 mg of calcium pantothenate, 1.75 mg of ferrous sulfate, 0.82 mg of zinc oxide, 25.3 mg of magnesium carbonate, 15 mg of monobasic potassium phosphate, 55 mg of dibasic calcium phosphate, 90 mg of potassium citrate, 100 mg of calcium carbonate and 24.8 mg of magnesium chloride. The mixing proportion may be changed differently. The mixture may be prepared into granules according to a method commonly used in the related art and may be used for the preparation of a health food composition according to a method commonly used in the related art.

### 2-2. Health drink

1,000 mg of the panduratin derivative of Examples 2 through 4 or the *Kaempferia pandurata* extract of Example 1 may be mixed with 1,000 mg of citric acid, 100 g of oligosaccharide, 2 g of plum extract and 1 g of taurine according to a method commonly used in the related art. Purified water may be added to a total volume of 900 mL. After heating at 85 °C for about 1 hour while stirring, the resultant solution may be filtered and collected in a sterilized 2 L container. After sealing and sterilization, it may be kept cold to prepare a health drink composition.

### 2-3. Chewing gum

0.1 wt% of the panduratin derivative of Examples 2 through 4 or the *Kaempferia pandurata* extract of Example 1 may be mixed with 20 wt% of gum base, 76.9 wt% of sugar, 1 wt% of perfume and 2 wt% of water according to a method commonly used in the related art to prepare chewing gum.

### 2-4. Candy

0.1 wt% of the panduratin derivative of Examples 2 through 4 or the *Kaempferia pandurata* extract of Example 1 may be mixed with 60 wt% of sugar, 39.8 wt% of starch syrup and 0.1 wt% of perfume according to a method commonly used in the related art to prepare candy.

### 2-5. Biscuit

1 wt% of the panduratin derivative of Examples 2 through 4 or the *Kaempferia pandurata* extract of Example 1 may be mixed with 25.59 wt% of hard wheat flour, 22.22 wt% of medium wheat flour, 4.80 wt% of refined sugar, 0.73 wt% of table salt, 0.78 wt% of glucose, 11.78 wt% of palm shortening, 1.54 wt% of ammonium, 0.17 wt% of baking soda, 0.16 wt% of sodium bisulfite, 1.45 wt% of rice flour, 0.0001 wt% of vitamin B₁, 0.0001 wt% of vitamin B₂, 0.04 wt% of milk flavor, 20.6998 wt% of water, 1.16 wt% of whole milk powder, 0.29 wt% of milk replacer, 0.03 wt% of monobasic calcium phosphate, 0.29 wt% of scattering salt and 7.27 wt% of spray-milk according to a method commonly used in the related art to prepare biscuit.

### Industrial Applicability

The said the panduratin derivative or the *Kaempferia pandurata* extract comprising the same induces cell proliferation, inhibits degradation of collagen, and promotes synthesis of collagen, therefore, it shows excellent activity in prevention of aging, particularly preventing or treating wrinkle. While it can be used as an effective ingredient in a cosmetic or food or nutritional supplement, these products are not claimed per se but only non-therapeutic uses of a cosmetic or food or nutritional supplement thereof.

## Claims

1. A non-therapeutic use of a cosmetic composition, a food or a nutritional supplement for preventing or treating wrinkles, or for preventing aging, wherein the composition, food or nutritional supplement promotes collagen synthesis, and comprises a panduratin derivative selected from the group consisting of the compounds represented by the following Chemical Formulas 1 to 3 or a *Kaempferia pandurata* extract comprising the panduratin derivative as an effective ingredient:

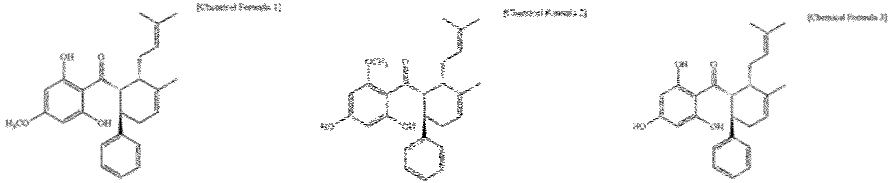

2. A non-therapeutic use of a cosmetic composition, a food or a nutritional supplement for preventing or treating wrinkles, or for preventing aging, wherein the composition, food or nutritional supplement inhibits collagen degradation, and comprises a panduratin derivative selected from the group consisting of the compounds represented by the following Chemical Formulas 1 to 3 or a *Kaempferia pandurata* extract comprising the panduratin derivative as an effective ingredient:

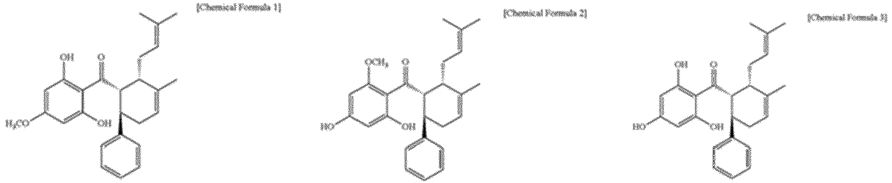

3. The non-therapeutic use of a cosmetic composition, a food or a nutritional supplement according to claim 1 or 2, wherein the composition, food or nutritional supplement is used to treat wrinkles caused by UV exposure.

4. The non-therapeutic use of a cosmetic composition, a food or a nutritional supplement according to claim 1 or 2, wherein the *Kaempferia pandurata* extract is extracted with a solvent which is selected from the group consisting of water, C1-C6 organic solvent and subcritical or supercritical fluid.

5. The non-therapeutic use of a cosmetic composition, a food or a nutritional supplement according to claim 4, wherein the C1-C6 organic solvent is one which is selected from the group consisting of C1-C6 alcohol, acetone, ether, benzene, chloroform, ethyl acetate, methylene chloride, hexane, cyclohexane, and petroleum ether.

6. A non-therapeutic method for promoting collagen synthesis using a panduratin derivative selected from the group consisting of the compounds represented by the following Chemical Formulas 1 to 3 or a *Kaempferia pandurata* extract comprising the panduratin derivative as an effective ingredient:

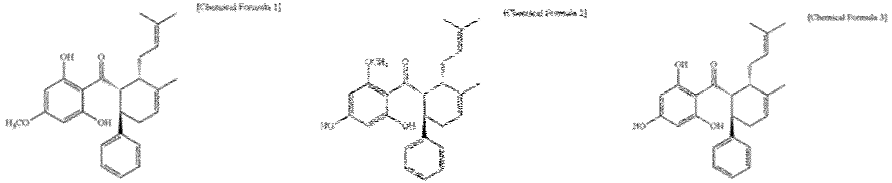

7. A non-therapeutic method for inhibiting collagen degradation using a panduratin derivative selected from the group consisting of the compounds represented by the following Chemical Formulas 1 to 3 or a *Kaempferia pandurata* extract comprising the panduratin derivative as an effective ingredient:

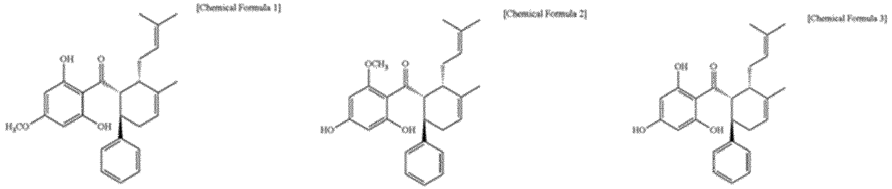

8. The non-therapeutic method according to claim 6 or 7, wherein the *Kaempferia pandurata* extract is extracted with a solvent which is selected from the group consisting of water, C1-C6 organic solvent and subcritical or supercritical fluid.

9. The non-therapeutic method according to claim 8, wherein the C1-C6 organic solvent is one which is selected from the group consisting of C1-C6 alcohol, acetone, ether, benzene, chloroform, ethyl acetate, methylene chloride, hexane, cyclohexane, and petroleum ether.

## Patentansprüche

1. Nicht-therapeutische Verwendung einer kosmetischen Zusammensetzung zur Prävention oder Behandlung von Falten, oder zur Prävention der Alterung, wobei die Zusammensetzung die Kollagensynthese fördert, und sie ein Panduratin-Derivat umfasst, ausgewählt aus der Gruppe, bestehend aus den Verbindungen, dargestellt durch die nachfolgenden chemischen Formeln 1 bis 3, oder einem Extrakt aus *Kaempferia pandurata,* umfassend das Panduratin-Derivat als wirksamen Bestandteil:

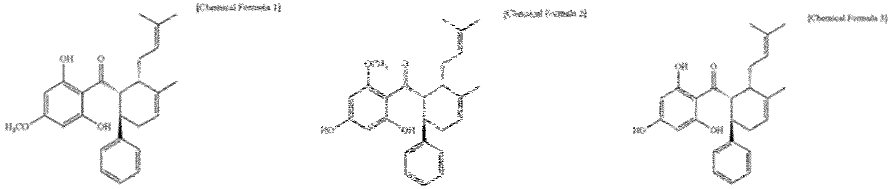

2. Nicht-therapeutische Verwendung einer kosmetischen Zusammensetzung zur Prävention oder Behandlung von Falten, oder zur Prävention der Alterung, wobei die Zusammensetzung den Kollagenabbau inhibiert, und sie ein Panduratin-Derivat umfasst, ausgewählt aus der Gruppe, bestehend aus den Verbindungen, dargestellt durch die nachfolgenden chemischen Formeln 1 bis 3, oder einem Extrakt aus *Kaempferia pandurata,* umfassend das Panduratin-Derivat als wirksamen Bestandteil:

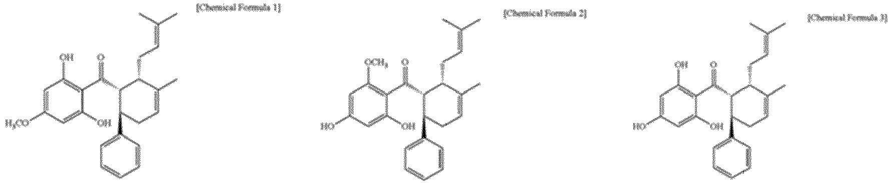

3. Nicht-therapeutische Verwendung einer kosmetischen Zusammensetzung nach Anspruch 1 oder 2, wobei die Zusammensetzung zur Behandlung von Falten eingesetzt wird, die durch UV-Exposition verursacht werden.

4. Nicht-therapeutische Verwendung einer kosmetischen Zusammensetzung nach Anspruch 1 oder 2, wobei der Extrakt aus *Kaempferia pandurata* mit einem Lösungsmittel extrahiert wird, welches ausgewählt wird aus der Gruppe, bestehend aus Wasser, einem C1-C6 organischen Lösungsmittel und subkritischer oder superkritischer Flüssigkeit.

5. Nicht-therapeutische Verwendung einer kosmetischen Zusammensetzung nach Anspruch 4, wobei das C1-C6 organische Lösungsmittel eines ist, welches ausgewählt wird aus der Gruppe, bestehend aus C1-C6 Alkohol, Aceton, Ether, Benzol, Chloroform, Ethylacetat, Methylenchlorid, Hexan, Cyclohexan, und Petrolether.

6. Nicht-therapeutisches Verfahren zur Förderung der Kollagensynthese unter Verwendung eines Panduratin-Derivats, ausgewählt aus der Gruppe, bestehend aus den Verbindungen, dargestellt durch die nachfolgenden chemischen Formeln 1-3 oder einem Extrakt aus *Kaempferia pandurata,* umfassend das Panduratin-Derivat als eine wirksame Verbindung:

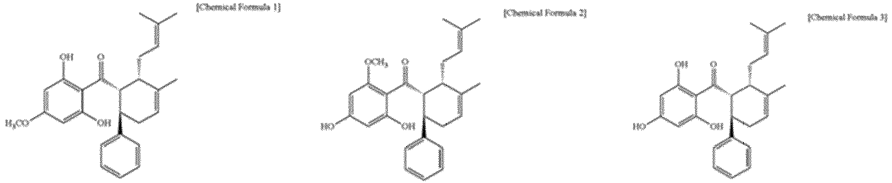

7. Nicht-therapeutisches Verfahren zur Inhibierung des Kollagenabbaus unter Verwendung eines Panduratin-Derivats, ausgewählt aus der Gruppe, bestehend aus den Verbindungen, dargestellt durch die nachfolgenden chemischen Formeln 1-3 oder einem Extrakt aus *Kaempferia pandurata,* umfassend das Panduratin-Derivat als eine wirksame Verbindung:

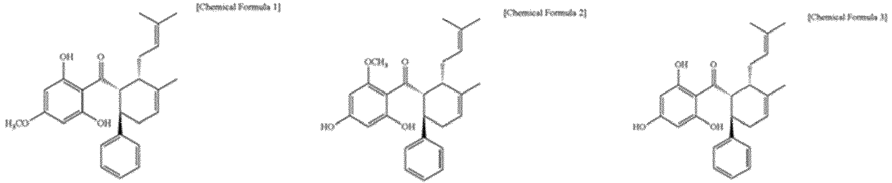

8. Nicht-therapeutisches Verfahren nach Anspruch 6 oder 7, wobei der Extrakt aus *Kaempferia pandurata* mit einem Lösungsmittel extrahiert wird, welches ausgewählt wird aus der Gruppe, bestehend aus Wasser, einem C1-C6 organischen Lösungsmittel und subkritischer oder superkritischer Flüssigkeit.

9. Nicht-therapeutisches Verfahren nach Anspruch 8, wobei das C1-C6 organische Lösungsmittel eines ist, welches ausgewählt wird aus der Gruppe, bestehend aus C1-C6 Alkohol, Aceton, Ether, Benzol, Chloroform, Ethylacetat, Methylenchlorid, Hexan, Cyclohexan, und Petrolether.

## Revendications

1. Utilisation non thérapeutique d'une composition cosmétique destinée à la prévention ou au traitement des rides, ou destinée à la prévention du vieillissement, dans laquelle la composition favorise la synthèse du collagène, et comprend un dérivé de panduratine choisi dans le groupe constitué par les composés représentés par les formules chimiques 1 à 3 suivantes ou un extrait de *Kaempferia pandurata* comprenant le dérivé de panduratine en tant que constituant efficace :

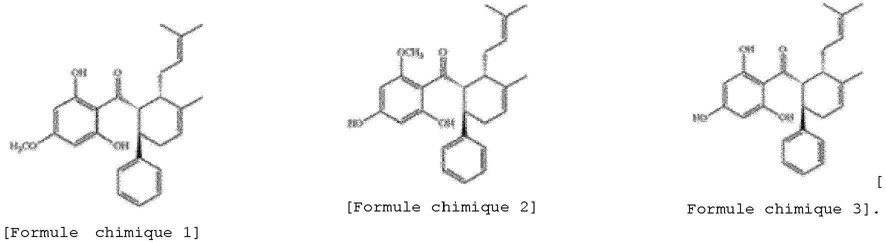

2. Utilisation non thérapeutique d'une composition cosmétique destinée à la prévention ou au traitement des rides, ou destinée à la prévention du vieillissement, dans laquelle la composition inhibe la dégradation du collagène, et comprend un dérivé de panduratine choisi dans le groupe constitué par les composés représentés par les formules chimiques 1 à 3 suivantes ou un extrait de *Kaempferia pandurata* comprenant le dérivé de panduratine en tant que constituant efficace :

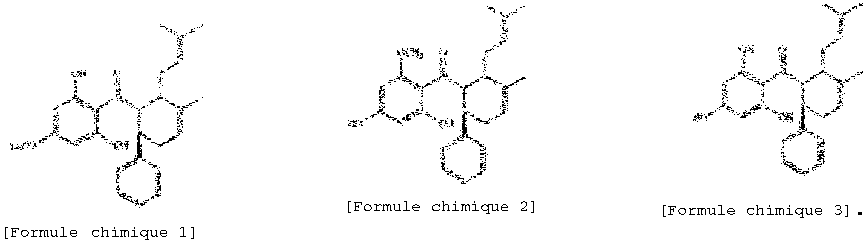

3. Utilisation non thérapeutique d'une composition cosmétique selon la revendication 1 ou la revendication 2, dans laquelle la composition est utilisée pour traiter les rides causées par l'exposition aux UV.

4. Utilisation non thérapeutique d'une composition cosmétique selon la revendication 1 ou la revendication 2, dans laquelle l'extrait de *Kaempferia pandurata* est extrait avec un solvant qui est choisi dans le groupe constitué par l'eau, un solvant organique en C1 à C6 ou un fluide subcritique ou supercritique.

5. Utilisation non thérapeutique d'une composition cosmétique selon la revendication 4, dans laquelle le solvant organique en C1 à C6 est un solvant qui est choisi dans le groupe constitué par les alcools en C1 à C6, l'acétone, l'éther, le benzène, le chloroforme, l'acétate d'éthyle, le chlorure de méthylène, l'hexane, le cyclohexane et l'éther de pétrole.

6. Procédé non thérapeutique destiné à favoriser la synthèse du collagène en utilisant un dérivé de panduratine choisi dans le groupe constitué par les composés représentés par les formules chimiques 1 à 3 suivantes ou un extrait de *Kaempferia pandurata* comprenant le dérivé de panduratine en tant que constituant efficace :

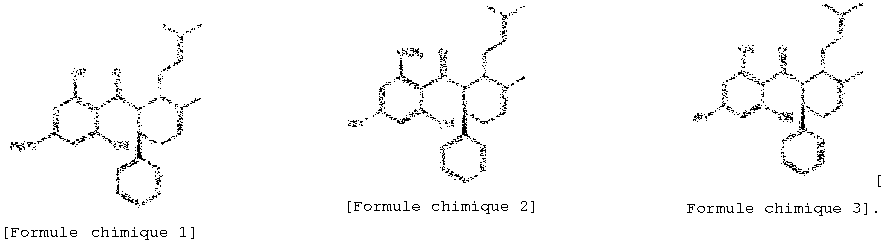

7. Procédé non thérapeutique destiné à inhiber la dégradation du collagène en utilisant un dérivé de panduratine choisi dans le groupe constitué par les composés représentés par les formules chimiques 1 à 3 suivantes ou un extrait de *Kaempferia pandurata* comprenant le dérivé de panduratine en tant que constituant efficace :

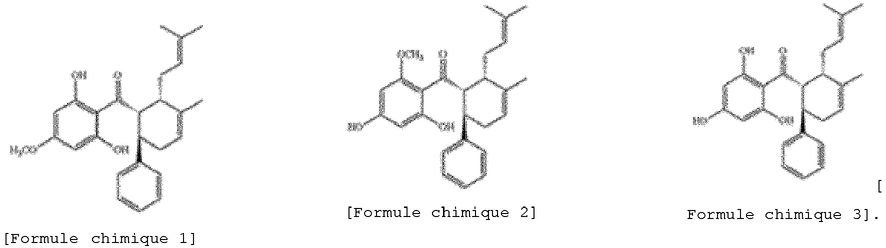

8. Procédé non thérapeutique selon la revendication 6 ou la revendication 7, dans lequel l'extrait de *Kaempferia pandurata* est extrait avec un solvant qui est choisi dans le groupe constitué par l'eau, un solvant organique en C1 à C6 ou un fluide subcritique ou supercritique.

9. Procédé non thérapeutique selon la revendication 8, dans lequel le solvant organique en C1 à C6 est un solvant qui est choisi dans le groupe constitué par les alcools en C1 à C6, l'acétone, l'éther, le benzène, le chloroforme, l'acétate d'éthyle, le chlorure de méthylène, l'hexane, le cyclohexane et l'éther de pétrole.
